# EUROPEAN PATENT SPECIFICATION

(11) **EP 4 133 348 B1**
(45) Date of publication and mention of the grant of the patent: **20.11.2024**
(21) Application number: 21783873.9
(22) Date of filing: 06.04.2021
(51) Int. Cl.: G06Q 10/00

(54) **METHODS FOR ARTIFICIAL POLLINATION AND APPARATUS FOR DOING THE SAME**
VERFAHREN ZUR KÜNSTLICHEN BESTÄUBUNG UND VORRICHTUNG ZUR DURCHFÜHRUNG DAVON
PROCÉDÉS DE POLLINISATION ARTIFICIELLE ET APPAREIL POUR LE FAIRE

(30) Priority: 06.04.2020 US 202063005677 P
(43) Date of publication of application: 15.02.2023
(73) Proprietor: BloomX Ltd., 4691500 Rishpon (IL)
(72) Inventor: ELGRABLI, Thai, 4691500 Rishpon (IL); SENESH, Ido, 3085500 Geva Carmel (IL); KEREN, Avi, 6962659 Tel-Aviv (IL)
(74) Representative: Lecomte & Partners
(86) International application number: PCT/IL2021/050386
(87) International publication number: WO 2021/205442

(56) References cited:
- WO-A1-2014/129966
- WO-A1-2019/049155
- WO-A1-2019/049155
- WO-A1-2019/158913
- CN-A- 108 243 733
- US-A1- 2016 334 276
- US-A1- 2016 353 661
- US-A1- 2016 353 661
- CHEN, YI ET AL.: "Intelligent autonomous pollination for future farming-a micro air vehicle conceptual framework with artificial intelligence and human-in-the-loop", IEEE ACCESS, vol. 7, 23 August 2019 (2019-08-23), pages 119706 - 119717, XP011743951, Retrieved from the Internet <URL:https://ieeexplore.ieee.org/abstract/document/8811448> DOI: 10.1109/ACCESS.2019.2937171

## Description

### TECHNICAL FIELD OF THE INVENTION

The invention relates to the field of artificial pollination. More specifically, the invention relates to pollen collecting element for pollination management.

### BACKGROUND OF THE INVENTION

Pollination is a process that involves transferring pollen grains from the male part of a plant to the female part of a plant (stigma). This process is done naturally by wind, small birds, insects (especially bees), and more. This process is crucial for later fertilization, production of seeds and the formation of fruits and vegetables. Disappearance of different natural pollinators, a phenomenon that if exacerbated, will severely harm the production of different crops that rely almost exclusively on insect pollination Moreover, the globalization process has shifted the production of different crops from their natural habitat into different geographies where the natural pollinators that evolved alongside the crop are not present. Their absence has a crucial effect on the quality of pollination, as the local pollinators may not be able to efficiently pollinate due to the inefficient pollen extraction, unsuitable body geometry and size, lack of attraction to the crop's nectar and pollen, which, altogether directly affect the crop's yield and quality. In addition, the insect-based pollination process depends on insect behavior, which could be tampered by weather conditions, temperatures, and other non-human controlled conditions.

Artificial pollination is a solution helping to overcome the difficulties mentioned above in a controlled and efficient way, thus providing an increase in crop yields and quality. Currently available solutions suffer from various deficiencies. For example, hand pollination is the simplest and cheapest artificial pollination method. Using different tools such as brushes one may gently collect pollen grains from a male flower and apply it directly to the female flower's stigma. Another method of hand pollination is rubbing the male organs of cut flowers on the female organs of the pollinated flowers. These methods are useful for a small scale, however, require a skilled human labor for applying on a large scale. Other solutions offer massive harvesting and processing of flowers to extract pollen grains. The collected pollen powder is then applied on the flowers with different techniques. This complicated process is both expensive and time-consuming, and it could not be performed in the field or orchard itself. This method is suitable for a certain type of crops with short-term mass flowering and a large number of pollen grains per flower.

US 2016/0353661 A1 discloses a method of pollinating a plant includes receiving, with a processing circuit, plant data regarding a plant having flowers, and controlling, by the processing circuit, operation of a robotic device to selectively pollinate a portion of the plurality of flowers based on the plant data. The robotic device includes sensors configured to acquire plant data, a pollination device configured to pollinate flowers of a plant, a collection device configured to collect pollen, and a pollination prevention device configured prevent a flower from being pollinated.

CN 108 243 733 A discloses a pollen collecting element specifically configured for collecting and treating pollen as food ingredient. To that end, it comprises a compartment with a collecting chamber, enclosing an electrostatic adsorber and a ultraviolet lamp. This pollen collecting element is not configured to collect pollen grains for release to artificially pollinate an agricultural area.

Providing scalable, in-field/in-orchard, cost-effective, well-controlled and easy-to-use systems and methods for artificial pollination, yield increase and in-field/in-orchard management remains a long and unmet need.

### SUMMARY OF THE INVENTION

The invention is directed to a pollen collecting element according to claim 1.

Further developments of the invention are according to dependent claims 2-12.

Additional features and advantages of the invention will become apparent from the following drawings and description.

### BRIEF DESCRIPTION OF THE DRAWINGS

**Fig.1** is a block diagram of an exemplary embodiment of a pollination management system;
**Fig.2** is a flowchart of an example of a pollination method;
**Fig.3A-C** is a flowchart of an example of a computer implemented pollination method;
**Fig.4A-B** is a graphic representation of the results of pollination on Avocado trees;
**Fig.5** is a schematic representation of an exemplary embodiment of a pollen collecting element; and
**Fig.6A-B** is a graphic representation of the results of pollination on Avocado trees.

### DETAILED DESCRIPTION OF THE INVENTION

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

According to some embodiments, the invention provides a pollination management system for an agricultural area comprising:
a) a pollination module, wherein said module comprises a pollen collecting element configured to collect the pollen; and a pollen application element configured to release the pollen;
b) a data acquisition module operably engaged with the pollination module, wherein said data acquisition module comprises at least one sensor;
c) a server in communication with the pollination module, and the data acquisition module, wherein the server is configured to process the data acquired by the data acquisition module;
d) an operation module comprising a controller in communication with the server and the pollination module, wherein said controller is configured to provide instructions to the pollination module; and,
e) a user interface;
wherein said system is configured to artificially pollinate the agricultural area or a desired portion of said agricultural area.

In one embodiment, the agricultural area is a field. In another embodiment, the agricultural area is an orchard. As used herein, the term "field" is meant to be understood as an area of cleared enclosed land used for cultivation crops. The list of crops of the invention includes without limitation crops such as maize, soybean, wheat, rice, oilseeds, cotton, grapes, sugarcane, fruits and vegetables. As used herein, the term orchard is meant to be understood as an area of land where crop trees are grown, while crop tree refers, without limitation, to trees that produce or have the potential to produce the desired produce. A non-limiting list of tree crops of the invention includes apple, avocado, nuts, cacao, kiwi, peach, pear, citrus such as orange, lemon, grapefruit, and tangerine; cherry, plum, apricot, mango, lychee, or any other tree crop that may gain benefit from the management system of the invention. As used herein, the term "portion" refers, without limitation, to a distinct part or a section of the whole, while "whole" is the entire agricultural area. The portion can be of any size, shape and geometry. It can be a large part of the area or a small part of the area. The size of the portion of the area can be pre-set, or maybe chosen sporadically.

According to some embodiments, the data acquisition module is configured to transmit the acquired data to the server. According to some embodiments, the instructions provided to the pollination module by the controller are based on the data processed by the server. In the context of the invention, the term "acquired data" is meant to be understood as data acquired by the data acquisition module by means of the at least one sensor and/or data received and/or gathered and/or transferred to and/or collected from an external source. Typically, the data acquired by the data acquisition module is a combination of the data obtained by the at least one sensor and data from an external source. The non-limiting list of data received from the external source include field related metadata, climate measurements and climate forecasting data, and field visual data. The unlimited list of field metadata may include plant types, planting maps, plants age, natural pollinators mapping beehives, and climate data. According to some embodiments, the climate data may either be obtained by 3^{rd} party service, from the client's weather station or using the at least one sensor of the data acquisition module. According to some embodiments, visual data could be either obtained by us using an external supplier, collected by image sensors of the data acquisition unit and/or drones and/or by 3^{rd} parties such as, without limitation, satellite imaging companies. In one embodiment, the instructions provided the to the pollination module by the controller are based on the data processed by the server. As used herein, the term "sensor" refers, without limitation to a device that detects and responds to some type of input from the physical environment. The specific input could be, without limitation, light, heat, motion, moisture, pressure, or any other environmental input. The non-limiting list of sensors of the invention includes one or more IR cameras, temperature sensor, humidity sensor, LIDAR, Sound recorder, GNSS, 4D imaging sensor, hyperspectral imaging, IMU, light sensor, or any other applicable sensor, or a combination thereof. In one embodiment, the data acquisition module comprises a plurality of sensors. In one embodiment, the data acquisition module comprises a plurality of sensors of the same type. In another embodiment, the data acquisitions module comprises a plurality of different sensors. In one embodiment, the data acquisition module comprises on-demand set of sensors. In one embodiment, the combination of different sensors is defined by the user. In one embodiment, the data acquisition module comprises a preset combination of sensors.

The invention provides a pollen collecting element of the pollination module, configured to collect pollen grains for release to artificially pollinate an agricultural area and comprising a high voltage power supply, an electrode, a pollen collection surface, and, optionally, a container. According to some embodiments, the pollen collecting element is configured to generate electric fields that apply electrostatic forces on the pollen grains thereby attracting the pollen grains to the pollen collection surface. The pollen collection surface comprises an outer surface of a covering of the electrode and the pollen collecting element exposes the pollen collection surface on an external portion of the pollen management system. In one embodiment, the pollen collecting element further comprises at least one of an internal control unit, a vibration engine, or a suction unit.

According to some embodiments, the server is configured to receive the acquired data, to store the acquired data, to generate a pollination heat map based on the acquired data, or any combination thereof. In one embodiment, the server further configured to transmit the pollination heat map to the operation module. In the context of the invention, the term "heat map" is meant to be understood, without limitation, as a holistic view on the field and/or orchard, pollination-wise, including, one or more of the following: identifying the flowering ratio in different parts of the fields, assessing pollination efficiency in areas based on environmental data, marking areas to be pollinated based on their needs and on previous pollinations activities and based on other environmental factors (weather, bees activities etc.).

According to some embodiments, the data are indicative of the crop status, such as flowering phenological and morphological stages, plant health and the environmental suitability for pollination. In one embodiment, the data comprise data are indicative of the pollination efficiency. As used herein, the term data refers, without limitation, to any information and/or input related to the state of the environment and the impacts on ecosystems. The data maybe acquired by the sensors of the data acquisition module, and/or can be gathered from external sources, such as, without limitation, weather forecast, activity of insects, or any other information that might be of relevance. The data acquired by the sensors may include, without limitation, health/disease state, yield estimations, flowering stage, and others.

According to some embodiments, the pollination management system of the invention is further configured to process real-time data acquired from the agricultural area and to pollinate the agricultural area based on said real-time data. In one embodiment, the real-time data are selected from the data acquired by the at least one sensor of the data acquisition module, data received by the data acquisition module from an external source, or a combination thereof. As used herein, the term "real-time" refers, without limitation to relating to a setting in which input data are processed within milliseconds so that it is available virtually immediately as feedback. In one embodiment, the real-time data are selected from the data acquired by the plurality of sensors, data collected from an external source, or a combination thereof.

According to some embodiments, the server is further configured to control at least one of: the area to be pollinated, pollination time, frequency of pollination episodes, number of pollination episodes, duration of each pollination episode, or any combination thereof. In one embodiment, the pollination of the agricultural area comprises more than one pollination episode. In one embodiment, the pollination of the agricultural area comprises a preset number of pollination episodes with predefined time intervals. In one embodiment, each pollination episode is performed based on the real-time data acquired by the data acquisition module. In one embodiment, the location of the area to be pollinated is determined based on the data and/or real-time data acquired by the data acquisition unit.

According to some embodiments of the invention, the pollen collecting element and the pollen application element form a single unit configured to collect and release the pollen. According to one embodiment, the pollen is collected using electrostatic forces applied to the pollen grains by the pollen collecting element, optionally, the pollen is stored in a container of a kind that is applicable for storage of pollen grains, and, at the desired timing, the pollen is released by the pollen application element directly and/or indirectly attached to the pollen collecting element, in such way, that the pollen collecting element and pollen application element together constitute a single assembly designed to both collect and release the pollen grains. According to some embodiments, the pollen collecting element and the pollen application element of the pollination module are separate elements. According to one embodiment, the pollen collecting element and the pollen application element of the pollination module are not attached, either directly or indirectly to each other, namely, the pollen grains are collected using electrostatic forces by the pollen collecting element, and then are stored at and/or transferred to the pollen application element of the pollination module.

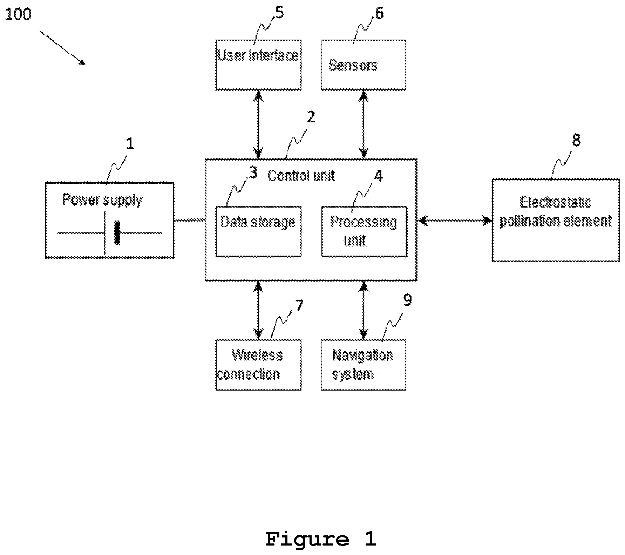

Reference is now made to Fig.1 illustrating a schematic diagram of an exemplary embodiment of a system 100 containing electrostatic pollination element 8 as part of the pollination module, and other components in order to optimize and improve the entire pollination process. The pollination module receives a pollination heat map from the central server in the field and/or orchard where the operator is located and navigates the operator to the relevant plants to be pollinated. The pollination element includes a power supply 1, control unit 2 with processing unit 4 is used to process real time data and to combine server inputs into the control of the pollination element and data storage 3 for storage all the data which received from the sensors 6. The sensors function is to collect environmental data (such as, without limitation, temperature, humidity, visible/non-visible light, and/or sound) about the plant status. The navigation system 9 with GNSS, mems and other navigation sensors can provide data on the current location and the inertial position of the worker on the tree. The user interface 5 includes a screen or instruction lights for guiding the operator where to do the pollination. A wireless connection 7 is used to send and receive data from the remote server/servers. According to some embodiments, the pollen collecting element further comprises at least one of the units selected from: an internal control unit, a vibration engine, and a suction unit.

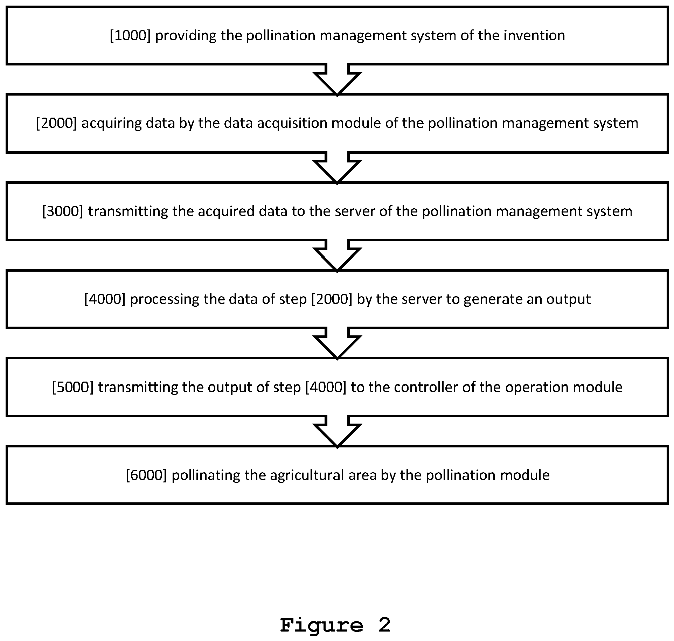

According to some examples, the invention provides a method of artificial pollination of an agricultural area in need of pollination. Reference is now made to Fig.2, illustrating a flow chart of an exemplary embodiment of the method of the invention. The method comprises: providing the pollination management system according to the embodiments of the invention [1000]; acquiring data by the data acquisition module of the pollination management system [2000]; transmitting the acquired data to the server of the pollination management system [3000]; transmitting the acquired data to the central server of the pollination management system [4000]; processing the data of step [2000] by the server to generate an output [5000]; transmitting the output of step [5000] to the controller of the operation module [6000], and, pollinating the agricultural area by the pollination module [7000]. In one embodiment, the step of pollinating the agricultural area is carried out according to the instructions provided by the controller. In one example, the instructions provided by the controller are based on the output of step [5000].

According to some examples of the above method, the step of pollinating the agricultural area comprises the steps of collecting the pollen by the pollen collecting element of the pollination module and releasing the pollen by the pollen application element of the pollination module.

According to some examples of the above method, the step of collecting the pollen by the pollen collecting element further comprises generating electric fields that apply electrostatic forces on the pollen grains thereby attracting the pollen grains to the pollen collection surface of the pollen collecting element.

According to some examples of the above method, the step of acquiring data by the data by the acquisition module comprises at least one of acquiring data by the at least one sensor of the data acquisition module, receiving data from an external source, or a combination thereof. In the context of the method of the invention the term "acquiring data" is meant to be understood as at least one action or series of actions performed by one or more components of the data acquisition module, that result in collection of data. The data can be acquired by that least one sensor of the data acquisition module and/or the data can be received from an external source. The data as well can be obtained by any other means, as long as the data are in a format suitable for the configuration of the data acquisition module. According to the embodiments of the system of the invention, data acquired by the at least one sensor of the pollination module may include, without limitation, location data (GPS), IMU data, visual data (e.g. images of crop plants while pollinating), climate related data such as temperature, humidity, wind etc.

These data will serve as a basis for measurements of both pollination effectiveness (e.g. assessing weather suitability for pollination, assess flowering and flowers receptiveness, operational needs of pollination - what was pollinated and when to later schedule additional activities). According to the embodiments data acquired by from an external source may include field related metadata, climate measurements and climate forecasting data, and field visual data. Field metadata could be plant types, planting maps, plants age, natural pollinators mapping beehives, climate data may either be obtained by 3^{rd} party service, from the client's weather station or using system's sensors.

According to some examples of the above method, the data a real-time data.

According to some examples of the above method, the step of processing the data and generating output comprises the steps selected from setting the number of pollination episodes, setting the timing of pollination, setting the frequency of pollination, setting the duration of pollination episode, setting the location of pollination, setting the area to be pollinated, or any combination thereof.

According to some examples of the above method, the timing of the pollination is set based on the acquired data. In one example, the location of the pollination is set based on the acquired data. In one example, the method comprises more than one pollination episode. In one example, the method comprises multiple pollination episodes. In one example, the method comprises a preset number of pollination episodes. In one example, the acquired data are real-time data, and pollination is a real-time pollination. In one example, the number of pollination episodes is set in real-time based on the real-time data. In one example, the pollination episodes are carried out with a preset frequency. In one example, each pollination episode has a preset duration.

According to some examples of the above method, the method further comprises repeating steps [2000] to [7000] predefined number of times.

According to some examples of the above method, the data are selected from the data acquired by the plurality of sensors of the data acquisition unit, data collected from at least one external source, or a combination thereof.

According to some example the agricultural area is a field of a crop. In one example, the agricultural area is an orchard.

According to some examples of the above method, the crop is a tree crop. In one example, the tree crop is selected, without limitation, from avocado, cocoa, nut, citrus, kiwi, peach, mango, lychee, pear, plum, cherry, apricot, or any other tree crop that may benefit from the pollination system and/or methods of the invention.

According to some examples of the above method, the crop is a field crop. In one example, the list of field crops of the invention includes, without limitation, crops such as maize, soybean, wheat, rice, oilseeds, cereals, cotton, grapes, sugarcane, fruits and vegetables.

According to some examples, the invention provides a method of increasing a yield of a crop comprising providing an artificial pollination management system, wherein said pollination management system comprises a pollination module; and, wherein said pollination module comprises at least one pollen collecting element; and, wherein said pollen collecting element is configured to apply electrostatic forces to the pollen grains, to thereby attract the pollen grains to the pollen collecting element. In one embodiment, the pollination management system further comprises a data acquisition module; a server in communication with the data acquisition module; an operation module in communication with the server, the data acquisition module and the pollination module; and a user interface. In one embodiment, the pollen grains are attracted to the pollen collection surface of the pollen collecting element. In one embodiment, the agricultural area is an orchard. In another embodiment, the agricultural area is field of a crop. As used herein, the term "yield" or "agricultural productivity" or "agricultural output" refers, without limitation, to the measure of the yield of a crop per unit area of land cultivation, and/or the seed generation of the plant itself. In the context of the invention, the increase in the yield can be measured according to any suitable parameter or technique known in the art and/or in-use by growers. For example, number of fruits and/or seeds per crop, size of the fruit, weight of the crop, and other parameters. According to some embodiments of the above method, the yield of the crop may be increased by 5% to 500%. In one embodiment, the yield of the crop may be increased by 10% to 500%. The yield of the crop maybe increased by 5%, 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%,260%, 270%, 280%, 290%, 300%, 310%, 320%, 330%, 340%, 350%,360%, 370%, 830%, 390%, 400%, 410%, 420%, 430%, 440%, 450%,460%, 470%, 480%, 490%, 500%.

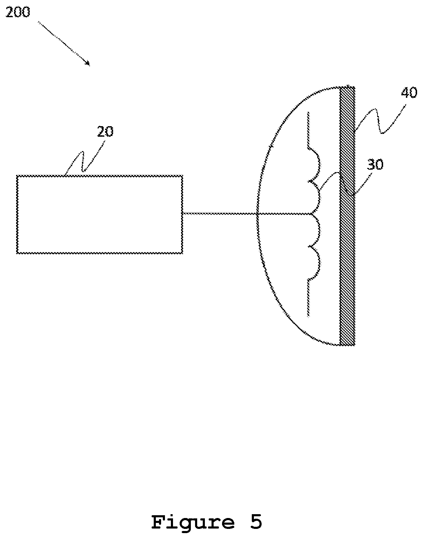

The invention provides a collecting element, configured to collect pollen grains for release to artificially pollinate an agricultural area, comprising a high voltage power supply, an electrode, a pollen collection surface, and, optionally, a container, wherein said high voltage power supply supplies high voltage to the electrode; and wherein said electrode is configured to generate electric fields that apply electrostatic forces on the pollen grains; and wherein said pollen grains are attracted to the pollen collecting surface of the element. The pollen collection surface comprises an outer surface of a covering of the electrode and the pollen collecting element exposes the pollen collection surface on an external portion of the pollen management system. In one embodiment, the pollen collecting element further comprises at least one of an internal control unit, a vibration engine, a blower, or a suction unit. Reference is now made to Fig.5, illustrating an exemplary embodiment of the pollen collecting element of the invention 200. A high voltage power supply 20 supplies high voltage to the electrode 30 which generates electric fields that apply electrostatic forces on the pollen grains. The pollen is then attracted to the pollen collection surface 40.

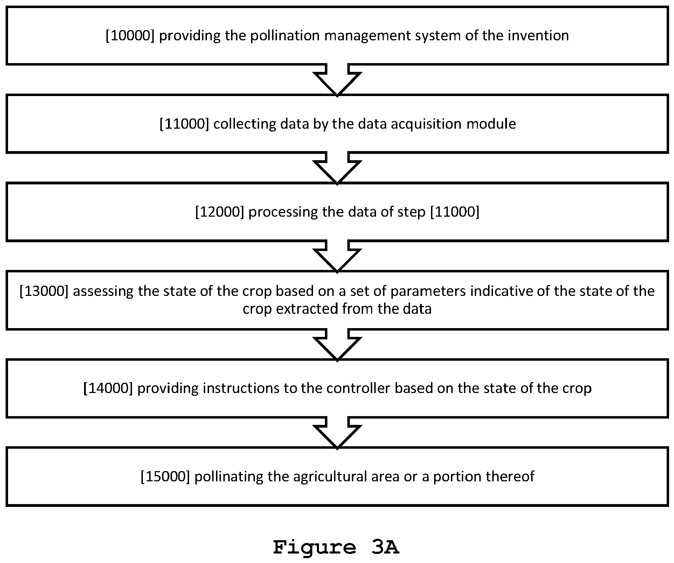

According to some examples, the invention provides a computer implemented method of artificial pollination of an agricultural area comprising a crop or a portion thereof. Reference is now made to Fig.3A, presenting a flowchart of an exemplary example of the method comprising the steps of: providing the artificial pollination system according to the examples of the invention [10000]; collecting data by the data acquisition module[11000]; processing the data of step [11000], [12000]; assessing the state of the crop based on a set of parameters indicative of the state of the crop extracted from the data [13000]; providing instructions to the controller based on the state of the crop [14000]; and, pollinating the agricultural area or a portion thereof [15000].

According to some examples of the above method, said data are image data.

According to some examples of the above method, said assessing the state of the crop comprises using a trained neural network. In one example, said step of processing comprises steps of computing said image data using computer implemented algorithm trained to generate output based on the image data. In another example, the computer implemented algorithm is trained to generate output based on predetermined feature vectors or attributes extracted from the image data. In one example, said method comprises steps of implementing with said algorithm a training process according to a training dataset comprising a plurality of training images of a plurality of crop plants captured by the at least one imaging sensor, wherein each respective training image of the plurality of training images is associated with the state of said crop plant depicted in the respective training image.

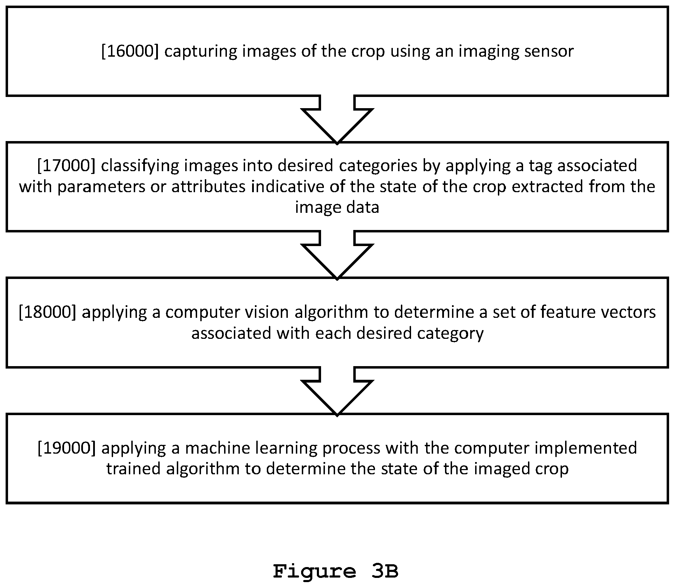

Reference is now made to Fig. 3B, representing an exemplary example of the above process wherein said training process comprises steps of capturing images of the crop plants using an imaging sensor [16000]; classifying images into desired categories by applying a tag associated with parameters or attributes indicative of the state of the crop extracted from the image data [17000]; and applying a computer vision algorithm to determine a set of feature vectors associated with each desired category [18000]. According to some examples, the above process further comprises the step of applying a machine learning process with the computer implemented trained algorithm to determine the state of the imaged crop [19000].

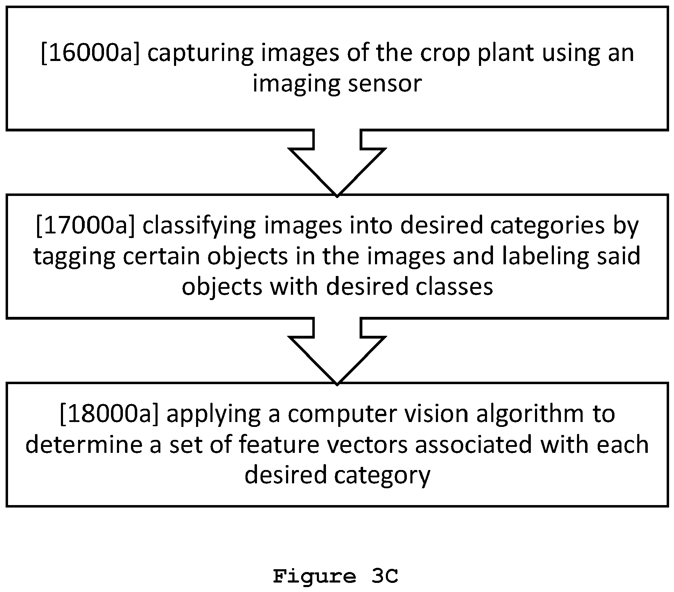

Reference is now made to Fig. 3C, representing an exemplary example of the above process wherein said training process comprises steps of capturing images of the crop plant using an imaging sensor [16000a]; classifying images into desired categories by tagging certain objects in the images and labeling said objects with desired classes [17000a]; and applying a computer vision algorithm to determine a set of feature vectors associated with each desired category [18000a].

According to some examples of the above method, the method comprises steps of applying a machine learning process with the computer implemented trained algorithm to determine the state of the imaged flowering plant. In one example, said algorithm is implemented with a machine learning process using a neural network with the processed data. In yet further example, said machine learning process comprises computing by the at least one neural network, a tag of at least one desired category for the at least one flowering plant, wherein the tag of at least one classification category is computed at least according to weights of the at least one neural network, wherein the at least one neural network is trained according to a training dataset comprising a plurality of training images of a plurality of crop plants captured by the at least one imaging sensor, wherein each respective training image of the plurality of training images is associated with said tag of at least one desired category of at least one crop plant depicted in the respective training image; and generating according to the tag of at least one classification category, instructions for execution by the controller. Alternatively, machine learning process comprises computing by the at least one neural network, a tag of at least one desired class for the at least one type of crop plant, wherein the tag of at least one class is computed at least according to weights of the at least one neural network, wherein the at least one neural network is trained according to a training dataset comprising a plurality of training images of a plurality of crop plants captured by the at least one imaging sensor, wherein each respective training image of the plurality of training images is associated with said tag of at least one desired class of at least one plant type depicted in the respective training image; and generating according to the tag of at least one class, instructions for execution by the controller.

In the context of the invention, the terms "crop" and/ "crop plant" are interchangeable and both meant to be understood as a whole plant and/or parts of the plant including, without limitation, leaves, fruits, seeds, stem, tree branch, tree trunk, flowers, or any other part not explicitly mentioned here. The crop plants of the invention are field crops and/or tree crops, so that the term "crop plant" includes tree or tree crop as well.

According to some examples, additional purpose of the invention is to disclose a holistic method for in-field/in-orchard pollination process: collection of pollen is performed using the pollen collecting element configured to apply electrostatic forces to the pollen grains; pollen application is performed using different methods such as, without limitation, electrostatic spraying, air pumps or any other application method of the art; data collected using the data acquisition module which is used to collect any data that will later be sent to a remote server using wireless or other connection. Those in the art will understand that a server may be a single computer, a network of computers, either in the cloud or on a local machine; the server will process the data and will provide insights to the grower; the server will provide recommendations about necessary future actions for the grower to take, using the device "user interface" (for real time actions) and using the dashboards (for real-time and/or offline actions). Those in the art will understand that serving the data could be supplied on different platforms, such as website, mobile application, tablet application and other platforms available in the market or under development. The insights and actions that the server may provide include, without limitation, pollination efficiency; recommendation on areas to re-pollinate artificially due to inefficient pollination; reporting on pests and physical damages in orchard/field; water condition; temperature and humidity near the crops, and any other parameter which may influence frequency and/or duration and/or location of pollination.

The terminology used herein is for the purpose of describing particular examples only and is not intended to be limiting of the invention.

According to some examples, the method of the present invention comprises steps of applying a machine learning process with the computer implemented trained algorithm to determine the state of the plant. Thus, it is within the scope of the present invention that the algorithm (or computer readable program) is implemented with a machine learning process using a neural network with the processed data. The term "training" in the context of machine learning implemented within the system of the present invention refers to the process of creating a machine learning algorithm. Training involves the use of a deep-learning framework and training dataset. A source of training data can be used to train machine learning models for a variety of use cases, from failure detection to consumer intelligence. The neural network may compute a classification category, and/or the embedding, and/or perform clustering, and/or detect objects from trained classes for identifying state of an individual plant in the context of pollination. As used herein the term "class" refers, without limitation, to a set or category of things having some property or attribute in common and differentiated from others by kind, type, or quality.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts.

As used herein, the term "classifying" may sometimes be interchanged with the term clustering or tagging, for example, when multiple plant images are analyzed, each image may be classified according to its predefined feature vectors and used to creating clusters, and/or the plant images may be embedded and the embeddings may be clustered. The term "desired category" may sometimes be interchanged with the term embedding, for example, the output of the trained neural network in response to an image of a plant may be one or more classification categories, or a vector storing a computed embedding. It is noted that the classification category and the embedding may be outputted by the same trained neural network, for example, the classification category is outputted by the last layer of the neural network, and the embedding is outputted by a hidden embedding layer of the neural network.

The architecture of the neural network(s) may be implemented, for example, as convolutional, pooling, nonlinearity, locally connected, fully connected layers, and/or combinations of the aforementioned.

It is noted that the tagging and classifying of the plants in the images or the plant state characteristic targets may be manually or semi manually entered by a user (e.g., via the GUI, for example, selected from a list of available phenotypic characteristic targets), obtained as predefined values stored in a data storage device, and/or automatically computed.

The term "feature vector" refers hereinafter in the context of machine learning to an individual measurable property or characteristic or parameter or attribute of a phenomenon being observed e.g., detected by a sensor. It is herein apparent that choosing an informative, discriminating and independent feature is a crucial step for effective algorithms in pattern recognition, machine learning, classification and regression. Algorithms using classification from a feature vector include nearest neighbor classification, neural networks, and statistical techniques. In computer vision and image processing, a feature is an information which is relevant for solving the computational task related to a certain application. Features may be specific structures in the image such as points, edges or objects. Features may also be the result of a general neighborhood operation or feature detection applied to the image. When features are defined in terms of local neighborhood operations applied to an image, a procedure commonly referred to as feature extraction is executed.

### Example 1: Cross-Pollination of Avocado Trees of Ettinger and Hass cultivars (one, three, six treatments)

40 trees were cross-pollinated between Ettinger and Hass cultivars. One, three or six consecutive treatments were performed vs the control group which had not received any artificial pollination treatments and average number of fruits and yield increase were measured. The results are represented in Table 1:

| Treatment | Average no. of Fruits | Yield Increase |
|---|---|---|
| One treatment | 219 | 52% |
| Three treatments | 318 | 121% |
| Six treatments | 250 | 73% |
| Control | 144 | 0 |

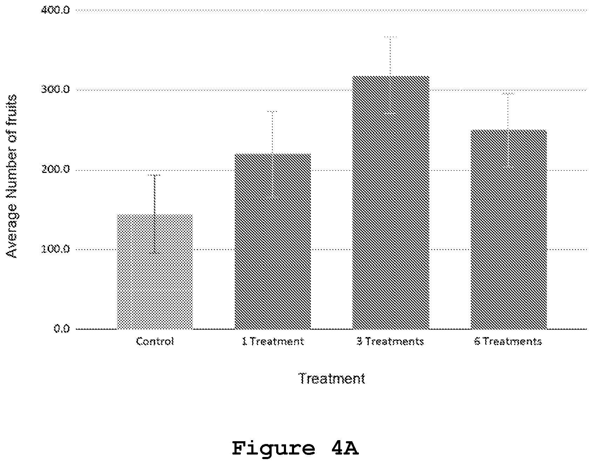

According to Table 1 and as represented on Fig. 4A, a significantly greater average number of fruits was observed as compared to the control group, and a significant increase in yield was evident.

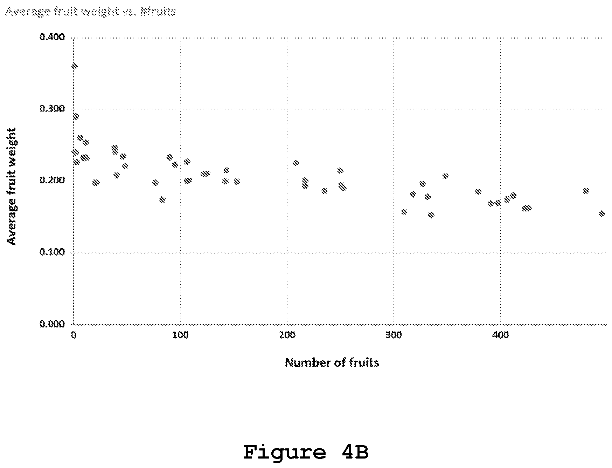

In addition, as shown on Fig. 4B, despite the increase in average no. of fruits, no change in fruit weight was observed.

### Example 2: Cross-Pollination of Avocado Trees of Ettinger and Hass cultivars (one, four, eight treatments)

40 trees were cross-pollinated between Ettinger and Hass cultivars. One, four or eight consecutive treatments were performed vs the control group which had not received any artificial pollination treatments and average number of fruits and yield increase were measured. The results are represented in Table 2:

| Treatment | Average no. of Fruits | Yield Increase |
|---|---|---|
| One treatment | 54.86 | 10.45% |
| Four treatments | 68.50 | 37.92 |
| Eight treatments | 79.25 | 59.56% |
| Control | 49.67 | 0 |

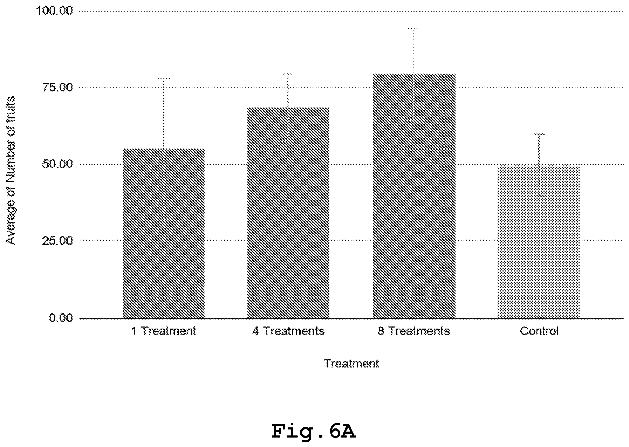

According to Table 2 and as represented on Fig. 6A, a greater average number of fruits was observed as compared to the control group, and a significant increase in yield was evident.

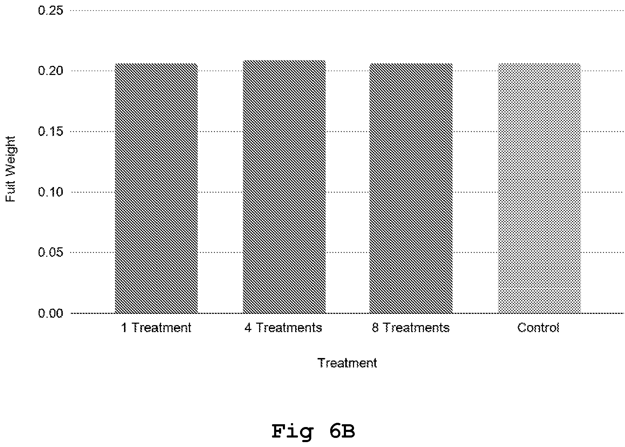

In addition, as shown on Fig. 6B, despite the increase in average no. of fruits, no change in average fruit weight per treatment was observed.

### Example 3: Artificial Pollination of Lychee Trees Litchi chinensis

40 lychee trees, are cross-pollinated between the Floridan variety (pollinator) and Mauritius variety (pollinated). Cross-pollination treatments are performed vs the control group which had not received any artificial pollination treatments and average number of fruits and yield increase are measured. Average fruit weight is measured as well. Other than the indicated treatments, the trees are grown at the same growth conditions.

An increase in average number of fruits and a significant yield increase are observed.

### Example 4: Artificial Pollination of Mango Trees Mangifera indica.

40 mango trees from varieties Tali, Keitt & Kent, are cross-pollinated or self-pollinated according to the treatments represented in Table 4:

| Tree id | Treatment | | | |
|---|---|---|---|---|
| 1 | Control | Tali x Tali | Keitt x Tali | Kent x Tali |
| 2 | Control | Tali x Tali | Keitt x Tali | Kent x Tali |
| 3 | Control | Tali x Tali | Keitt x Tali | Kent x Tali |
| 4 | Control | Tali x Tali | Keitt x Tali | Kent x Tali |
| 5 | Control | Tali x Tali | Keitt x Tali | Kent x Tali |
| 6 | Control | Tali x Tali | Keitt x Tali | Kent x Tali |
| 7 | Control | Tali x Tali | Keitt x Tali | Kent x Tali |
| 8 | Control | Tali x Tali | Keitt x Tali | Kent x Tali |
| 9 | Control | Tali x Tali | Keitt x Tali | Kent x Tali |
| 10 | Control | Tali x Tali | Keitt x Tali | Kent x Tali |

Treatments are performed vs the control group which had not received any artificial pollination treatments and average number of fruits and yield increase are measured. Average fruit weight is measured as well. Other than the indicated treatments, the trees are grown at the same growth conditions.

An increase in average number of fruits and a significant yield increase are observed.

The terminology used herein is for the purpose of describing particular embodiments only and is not intended to be limiting of the invention. As used herein, the singular forms "a," "an" and "the" are intended to include plural forms as well, unless the context clearly indicates otherwise. It will be further understood that the terms "comprises" or "comprising," when used in this specification, specify the presence of stated features, integers, steps, operations, elements components and/or groups or combinations thereof, but do not preclude the presence or addition of one or more other features, integers, steps, operations, elements, components and/or groups or combinations thereof. As used herein the terms "comprises", "comprising", "includes", "including", "having" and their conjugates mean "including but not limited to". The term "consisting of" means "including and limited to".

As used herein, the term "and/or" includes any and all possible combinations or one or more of the associated listed items, as well as the lack of combinations when interpreted in the alternative ("or").

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and claims and should not be interpreted in an idealized or overly formal sense unless expressly so defined herein. Well-known functions or constructions may not be described in detail for brevity and/or clarity.

It will be understood that when an element is referred to as being "on," "attached" to, "operatively coupled" to, "operatively linked" to, "operatively engaged" with, "connected" to, "coupled" with, "contacting," etc., another element, it can be directly on, attached to, connected to, operatively coupled to, operatively engaged with, coupled with and/or contacting the other element or intervening elements can also be present. In contrast, when an element is referred to as being "directly contacting" another element, there are no intervening elements present. Whenever the term "about" is used, it is meant to refer to a measurable value such as an amount, a temporal duration, and the like, and is meant to encompass variations of ±20%, ±10%, ±5%, ±1%, or ±0.1% from the specified value, as such variations are appropriate to perform the disclosed methods.

It will be understood that, terms such as, for example, "processing", "computing", "calculating", "determining", "establishing", "analyzing", "checking", or the like, may refer to operation(s) and/or process(es) of a computer, a computing platform, a computing system, or other electronic computing device, that manipulates and/or transforms data represented as physical (e.g., electronic) quantities within the computer's registers and/or memories into other data similarly represented as physical quantities within the computer's registers and/or memories or other information non-transitory storage medium that may store instructions to perform operations and/or processes.

It will be understood that, although the terms first, second, etc., may be used herein to describe various elements, components, regions, layers and/or sections, these elements, components, regions, layers and/or sections should not be limited by these terms. Rather, these terms are only used to distinguish one element, component, region, layer and/or section, from another element, component, region, layer and/or section.

Certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Whenever terms "plurality" and "a plurality" are used it is meant to include, for example, "multiple" or "two or more". The terms "plurality" or "a plurality" may be used throughout the specification to describe two or more components, devices, elements, units, parameters, or the like. The term set when used herein may include one or more items. Unless explicitly stated, the method examples described herein are not constrained to a particular order or sequence. Additionally, some of the described method examples or elements thereof can occur or be performed simultaneously, at the same point in time, or concurrently. All publications, patent applications, patents, and other references mentioned. In case of conflict, the patent specification, including definitions, will prevail. In addition, the materials, methods, and examples are illustrative only and not intended to be limiting. Throughout this application various publications, published patent applications and published patents are referenced.

It will be appreciated by persons skilled in the art that the present invention is not limited to what has been particularly shown and described hereinabove. Rather the scope of the present invention is defined by the appended claims and includes both combinations and sub-combinations of the various features described hereinabove as well as variations and modifications thereof, which would occur to persons skilled in the art upon reading the foregoing description.

## Claims

1. A pollen collecting element (200) of a pollen management system (100) configured to collect pollen grains for release to artificially pollinate an agricultural area, the pollen collecting element comprising:
a high voltage power supply (20);
an electrode (30); and
a pollen collection surface (40);
wherein:
said high voltage power supply (20) supplies high voltage to the electrode (30), and
said electrode (30) is configured to generate electric fields that apply electrostatic forces on the pollen grains, and
said pollen grains are attracted to the pollen collecting surface (40) of the pollen collecting element (20) ;
**characterized in that**:
the pollen collection surface (40) comprises an outer surface of a covering of the electrode (30); and
the pollen collecting element (200) exposes the pollen collection surface (40) on an external portion of the pollen management system (100).

2. The pollen collecting element (200) of claim 1, wherein the pollen collection surface (40) comprises a flat surface region.

3. The pollen collecting element (200) of claim 1, together with the pollen management system (100) operating as a pollination module, and further comprising:
a pollen application element of the pollination module, configured to release the pollen;
a data acquisition module operably engaged with the pollination module, wherein said data acquisition module comprises at least one sensor;
a server in communication with the pollination module, and the data acquisition module, wherein the server is configured to process the data acquired by the data acquisition module;
an operation module comprising a controller in communication with the server and the pollination module, wherein said controller is configured to provide instructions to the pollination module; and
a user interface.

4. The pollen collecting element (200) and pollen management system (100) of claim 3, wherein the data acquisition module is configured to transmit the acquired data to the server; and, wherein the instructions provided the to the pollination module by the controller are based on the data processed by the server

5. The pollen collecting element (200) and pollen management system (100) of one of claims 3 and 4, wherein the at least one sensor is selected from the group consisting of IR camera, temperature sensor, humidity sensor, LIDAR, Sound recorder, GNSS, 4D imaging sensor, hyperspectral imaging, IMU, and a light sensor.

6. The pollen collecting element (200) and pollen management system (100) of any one of claims 3 to 5, wherein the server is configured to receive the acquired data, to store the acquired data, to generate a pollination heat map based on the acquired data.

7. The pollen collecting element (200) and pollen management system (100) of claim 6, wherein the server further configured to transmit the pollination heat map to the operation module.

8. The pollen collecting element (200) and pollen management system (100) of any one of claims 3 to 7, wherein the data are selected from location data (GPS), IMU data, visual data, and climate related data; and wherein the data are real-time data selected from the data acquired by the at least one sensor of the data acquisition module.

9. The pollen collecting element (200) and pollen management system (100) of any one of claims 3 to 8, wherein the server is further configured to control at least one of: an area to be pollinated, pollination time, frequency of pollination episodes, number of pollination episodes, and duration of each pollination episode.

10. The pollen collecting element (200) and pollen management system (100) of any one of claims 3 to 9, wherein the agricultural area is a field of a crop or an orchard.

11. The pollen collecting element (200) of claim 1, further comprising at least one of an internal control unit, a vibration engine, a blower, or a suction unit.

12. The pollen collecting element (200) of claim 1, wherein the electrode (30) extends along the pollen collecting surface (40).

## Patentansprüche

1. Ein Pollensammelelement (200) eines Pollenmanagementsystems (100), das so gestaltet ist, dass es Pollenkörner zur Freisetzung sammelt, um ein landwirtschaftliches Gebiet künstlich zu bestäuben, wobei das Pollensammelelement Folgendes umfasst:
eine Hochspannungsstromversorgung (20);
eine Elektrode (30); und
eine Pollensammelfläche (40); wobei:
die Hochspannungsstromversorgung (20) die Elektrode (30) mit Hochspannung versorgt, und
die Elektrode (30) so gestaltet ist, dass sie elektrische Felder erzeugt, die elektrostatische Kräfte auf die Pollenkörner ausüben, und
die Pollenkörner von der Pollensammelfläche (40) des Pollensammelelements (2D) angezogen werden;
**dadurch gekennzeichnet, dass**:
die Pollensammelfläche (40) eine Außenfläche einer Abdeckung der Elektrode (30) umfasst; und
das Pollensammelelement (200) die Pollensammelfläche (40) an einem äußeren Teil des Pollenmanagementsystems (100) freilegt.

2. Das Pollensammelelement (200) nach Anspruch 1, wobei die Pollensammelfläche (40) einen flachen Oberflächenbereich aufweist.

3. Das Pollensammelelement (200) nach Anspruch 1, das zusammen mit dem Pollenmanagementsystem (100) als Bestäubungsmodul arbeitet, und das ferner umfasst:
ein Pollenausbringungselement des Bestäubungsmoduls, das so gestaltet ist, dass es den Pollen freisetzt;
ein Datenerfassungsmodul, das funktionsfähig mit dem Bestäubungsmodul verbunden ist, wobei das Datenerfassungsmodul mindestens einen Sensor umfasst;
einen Server, der mit dem Bestäubungsmodul und dem Datenerfassungsmodul verbunden ist, wobei der Server so gestaltet ist, dass er die von dem Datenerfassungsmodul erfassten Daten verarbeitet;
ein Betriebsmodul, das eine Steuerung umfasst, die mit dem Server und dem Bestäubungsmodul in Verbindung steht, wobei die Steuerung so ausgelegt ist, dass sie Anweisungen für das Bestäubungsmodul bereitstellt; und
eine Benutzerschnittstelle.

4. Das Pollensammelelement (200) und Pollenmanagementsystem (100) nach Anspruch 3, wobei das Datenerfassungsmodul so gestaltet ist, dass es die erfassten Daten an den Server überträgt; und wobei die Anweisungen, die dem Bestäubungsmodul von der Steuerung bereitgestellt werden, auf den vom Server verarbeiteten Daten beruhen.

5. Das Pollensammelelement (200) und Pollenmanagementsystem (100) nach einem der Ansprüche 3 und 4, wobei der mindestens eine Sensor aus der Gruppe ausgewählt ist, die aus einer IR-Kamera, einem Temperatursensor, einem Feuchtigkeitssensor, einem LIDAR, einem Tonaufzeichnungsgerät, einem GNSS, einem 4D-Bildsensor, einer hyperspektralen Bildgebung, einer IMU und einem Lichtsensor besteht.

6. Das Pollensammelelement (200) und Pollenmanagementsystem (100) nach einem der Ansprüche 3 bis 5, wobei der Server so gestaltet ist, dass er die erfassten Daten empfängt, die erfassten Daten speichert und eine Bestäubungswärmekarte auf der Grundlage der erfassten Daten erzeugt.

7. Das Pollensammelelement (200) und Pollenmanagementsystem (100) nach Anspruch 6, wobei der Server ferner so gestaltet ist, dass er die Bestäubungswärmekarte an das Betriebsmodul übermittelt.

8. Das Pollensammelelement (200) und Pollenmanagementsystem (100) nach einem der Ansprüche 3 bis 7, wobei die Daten aus Standortdaten (GPS), IMU-Daten, visuellen Daten und klimabezogenen Daten ausgewählt sind; und wobei die Daten Echtzeitdaten sind, die aus den von dem mindestens einen Sensor des Datenerfassungsmoduls erfassten Daten ausgewählt sind.

9. Das Pollensammelelement (200) und Pollenmanagementsystem (100) nach einem der Ansprüche 3 bis 8, wobei der Server ferner so gestaltet ist, dass er mindestens eines der folgenden Elemente steuert: ein zu bestäubendes Gebiet, die Bestäubungszeit, die Häufigkeit von Bestäubungsepisoden, die Anzahl von Bestäubungsepisoden und die Dauer jeder Bestäubungsepisode.

10. Das Pollensammelelement (200) und Pollenmanagementsystem (100) nach einem der Ansprüche 3 bis 9, wobei die landwirtschaftliche Fläche ein Feld einer Kulturpflanze oder ein Obstgarten ist.

11. Das Pollensammelelement (200) nach Anspruch 1 umfasst ferner mindestens eine interne Steuereinheit, einen Vibrationsmotor, ein Gebläse oder eine Saugeinheit.

12. Das Pollensammelelement (200) nach Anspruch 1, wobei sich die Elektrode (30) entlang der Pollensammelfläche (40) erstreckt.

## Revendications

1. Un élément de collecte de pollen (200) d'un système de gestion du pollen (100) configuré pour collecter des grains de pollen afin de les libérer pour polliniser artificiellement une zone agricole, l'élément de collecte de pollen comprenant :
une alimentation haute tension (20) ;
une électrode (30) ; et
une surface de collecte de pollen (40) ;
dans lequel :
ladite alimentation haute tension (20) fournit une haute tension à l'électrode (30), et
ladite électrode (30) est configurée pour générer des champs électriques qui appliquent des forces électrostatiques sur les grains de pollen, et
lesdits grains de pollen sont attirés par la surface de collecte de pollen (40) de l'élément de collecte de pollen (20) ;
**caractérisé en ce que** :
la surface de collecte de pollen (40) comprend une surface extérieure d'un revêtement de l'électrode (30) ; et
l'élément de collecte de pollen (200) expose la surface de collecte de pollen (40) sur une partie externe du système de gestion du pollen (100).

2. L'élément de collecte de pollen (200) selon la revendication 1, dans lequel la surface de collecte de pollen (40) comprend une zone de surface plate.

3. L'élément de collecte de pollen (200) selon la revendication 1, associé au système de gestion du pollen (100) fonctionnant comme un module de pollinisation, et comprenant en outre :
un élément d'application du pollen du module de pollinisation, configuré pour libérer le pollen ;
un module d'acquisition de données fonctionnellement engagé avec le module de pollinisation, ledit module d'acquisition de données comprenant au moins un capteur ;
un serveur en communication avec le module de pollinisation et le module d'acquisition de données, le serveur étant configuré pour traiter les données acquises par le module d'acquisition de données ;
un module d'exploitation comprenant un contrôleur en communication avec le serveur et le module de pollinisation, ledit contrôleur étant configuré pour fournir des instructions au module de pollinisation ; et
une interface utilisateur.

4. L'élément de collecte de pollen (200) et le système de gestion de pollen (100) selon la revendication 3, dans lequel le module d'acquisition de données est configuré pour transmettre les données acquises au serveur ; et, dans lequel les instructions fournies au module de pollinisation par le contrôleur sont basées sur les données traitées par le serveur.

5. L'élément de collecte de pollen (200) et le système de gestion du pollen (100) selon l'une quelconque des revendications 3 et 4, dans lequel ledit au moins un capteur est choisi dans le groupe constitué par une caméra IR, un capteur de température, un capteur d'humidité, un LIDAR, un enregistreur sonore, un GNSS, un capteur d'imagerie 4D, une imagerie hyperspectrale, un IMU et un capteur de lumière.

6. L'élément de collecte de pollen (200) et le système de gestion du pollen (100) selon l'une quelconque des revendications 3 à 5, dans lequel le serveur est configuré pour recevoir les données acquises, pour stocker les données acquises, pour générer une carte thermique de pollinisation sur la base des données acquises.

7. L'élément de collecte de pollen (200) et le système de gestion du pollen (100) selon la revendication 6, dans lequel le serveur est en outre configuré pour transmettre la carte thermique de pollinisation au module d'exploitation.

8. L'élément de collecte de pollen (200) et le système de gestion du pollen (100) selon l'une quelconque des revendications 3 à 7, dans lequel les données sont sélectionnées parmi les données de localisation (GPS), les données IMU, les données visuelles et les données climatiques ; et dans lequel les données sont des données en temps réel sélectionnées parmi les données acquises par au moins un capteur du module d'acquisition de données.

9. L'élément de collecte de pollen (200) et le système de gestion du pollen (100) selon l'une quelconque des revendications 3 à 8, dans lequel le serveur est en outre configuré pour contrôler au moins l'un des éléments suivants : une zone à polliniser, le temps de pollinisation, la fréquence des épisodes de pollinisation, le nombre d'épisodes de pollinisation et la durée de chaque épisode de pollinisation.

10. L'élément de collecte de pollen (200) et le système de gestion du pollen (100) selon l'une quelconque des revendications 3 à 9, dans lequel la zone agricole est un champ de culture ou un verger.

11. L'élément de collecte de pollen (200) selon la revendication 1, comprenant en outre au moins l'un des éléments suivants : une unité de commande interne, un moteur à vibrations, un ventilateur ou une unité d'aspiration.

12. L'élément de collecte de pollen (200) selon la revendication 1, dans lequel l'électrode (30) s'étend le long de la surface de collecte de pollen (40).
